(19) Europäisches Patentamt
European Patent Office
Office européen des brevets

(11) Publication number: **0 108 547**
**B1**

(12) # EUROPEAN PATENT SPECIFICATION

(45) Date of publication of patent specification: **07.06.89**

(51) Int. Cl.⁴: **C 07 C 69/96**, C 07 D 499/08, C 07 D 499/32, A 61 K 31/43

(21) Application number: **83306482.7**

(22) Date of filing: **25.10.83**

(54) **Process for the preparation of the 1′-ethoxycarbonyloxyethyl ester of benzylpenicillin.**

(30) Priority: **07.01.83 IL 67637**
**04.11.82 IL 67177**

(43) Date of publication of application:
**16.05.84 Bulletin 84/20**

(45) Publication of the grant of the patent:
**07.06.89 Bulletin 89/23**

(84) Designated Contracting States:
**AT BE CH DE FR GB IT LI NL SE**

(56) References cited:
**BE-A- 897 161**
**GB-A-1 003 479**
**US-A-3 697 507**

**CHEMICAL ABSTRACTS, vol. 81, no. 19, 11th November 1974, page 543, no. 120666r, Columbus, Ohio, US**

**CHEMICAL ABSTRACTS, vol. 93, no. 23, 8th December 1980, page 531, no. 220736e, Columbus, Ohio, US**

**The file contains technical information submitted after the application was filed and not included in this specification**

(73) Proprietor: **ASTRA LAKEMEDEL AKTIEBOLAG**
**S-151 85 Södertälje (SE)**

(72) Inventor: **Vaya, Jacob**
**25 Horkania Street**
**Jerusalem 93 305 (IL)**
Inventor: **Kaspi, Joseph**
**13 Borochov Street**
**Givatayim 53 201 (IL)**
Inventor: **Ladkani, David**
**24 Shechunat Pat**
**Jerusalem (IL)**
Inventor: **Salemnick, Gad**
**14 Uziel Street**
**Jerusalem (IL)**
Inventor: **Schoenberger, Clara**
**17 Guatemalastreet**
**Jerusalem 96 704 (IL)**
Inventor: **Yellin, Haim**
**45 Hayarden Street**
**Ramat-Gan 52 271 (IL)**
Inventor: **Cherkez, Stephen**
**17 Shilo Street**
**Ramat-Gan 52 455 (IL)**

(74) Representative: **Hjertman, Ivan T. et al**
**AB ASTRA Patent and Trade Mark Department**
**S-151 85 Södertälje (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 108 547 B1**

**Description**

The present invention relates to a process for the preparation of the 1′-ethoxy carbonyloxy ethyl ester of penicillin G, in which a new esterification agent, 1-bromoethyl ethyl carbonate is used. The said ester of penicillin G is produced in better yields and higher purity than known before.

1-Chloroethyl ethyl carbonate is a known compound and has been used for esterification purposes in the preparation of drugs of the penicillin series such as, for example, penicillin G. Thus, for example, British Patent Specification No. 1,363,506 discloses esterification of penicillin G with 1-chloroethyl ethyl carbonate. The 1′-ethoxycarbonyloxy ethyl ester of penicillin G is an intermediate in the preparation of i.a. the antibacterial drug bacampicillin.

Bacampicillin, which is the 1′-ethoxy carbonyloxy ethyl ester of ampicillin (marketed as the hydrochloride) (formula I):

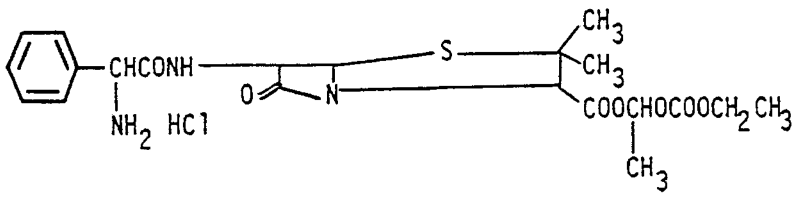

I

is being successfully used as an antibacterial drug. It is characterized by an almost quantitative absorption from the digestive tract to the blood stream, and a good tolerance by the gastro-intestinal system. Bacampicillin was first disclosed in said British Patent No. 1,363,506.

According to prior disclosures, bacampicillin was prepared in several ways.

For example, according to British patent No. 1,363,506 1′-ethoxycarbonyloxyethyl benzyl penicillinate was prepared by reacting potassium benzyl penicillinate with 1-chloroethyl ethyl carbonate in aqueous dioxane at room temperature for 66 hours. The ester was obtained at 33% yield with an unspecified degree of purity. An improvement was reported later in British patent No. 1,443,738 according to which the rather expensive tetrabutyl ammonium benzyl penicillinate was prepared and this salt was then subjected to the esterification with 1-chloroethyl ethyl carbonate in boiling acetone for 6 hours. The ester was obtained in an overall yield of 65% and in 90% purity.

The 1′-ethoxycarbonyloxy ethyl ester of penicillin G is then used in known manner as described e.g. in said British patent No. 1,363,506 as an intermediate in the preparation of i.a. bacampicillin.

However, the rate of esterification of benzylpenicillin with 1-chloroethyl ethyl carbonate is slow. Moreover, drastic conditions such as long reaction time, high temperature and the use of catalysts such as phase transfer catalysts are required. Where such conditions are applied to esterifications of sensitive compounds such as, for example, penicillins, they may cause partial degradation of the sensitive β-lactam system. In all the known processes for preparing bacampicillin, the yields are relatively low and many procedures give material of inferior quality.

The present invention provides a process for the preparation of the 1′-ethoxycarbonyloxy ethyl ester of penicillin G by which the said ester of penicillin G is obtained in very much higher yield, and with a higher degree of purity, than is obtained with prior art processes. Thus, the process of the invention is characterized in that benzyl penicillin, or a salt thereof, is reacted with 1-bromo ethyl ethyl carbonate in a non-hydroxylic polar solvent at a temperature of 20°C to 50°C. The process of the invention requires less reagent, shorter reaction time, lower reaction temperature and no catalyst while at the same time producing better yields. These advantages result also in a favourable effect on the yield of the desired final product.

The esterification according to the invention, of benzyl penicillin, suitably in the form of its potassium salt, is carried out in a non-hydroxylic polar solvent. Examples are acetone, 2-butanone, dimethylsulfoxide and acetonitrile. The preferred solvent is acetone. The reaction is best carried out in the presence of an acid absorber such as sodium bicarbonate. The temperature range for the reaction is between 20° to 50° but the range of 35—45° is preferred. Reaction is best carried out in moist solvent (ca. 4% water) in order to accelerate the esterification (enhancing the solubility of the potassium salt). The reaction rate can also be accelerated by the addition of a catalytic amount of a tertiary amino or quaternary ammonium salt such as triethylamine or tetrabutyl ammonium bromide, but the addition of such a salt is not an essential part of the invention.

Thus, potassium benzyl penicillinate was reacted with 1-bromoethyl ethyl carbonate in moist acetone at 40°C for 5 hours to give 1′-ethoxycarbonyloxy ethyl benzyl penicillinate in a quantitative yield and 99% purity. This is a dramatic increase in yield and product quality using at the same time milder reaction conditions as shown in accordance with the process of the present invention.

The present invention also relates to the use of 1-bromo ethyl ethyl carbonate in the said process for the preparation of the 1′-ethoxycarbonyloxy ethyl ester of penicillin G.

The chemical formulas are as follows:

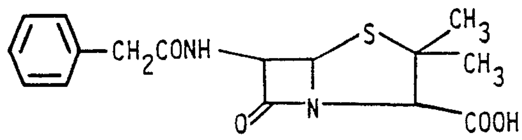

benzyl penicillin

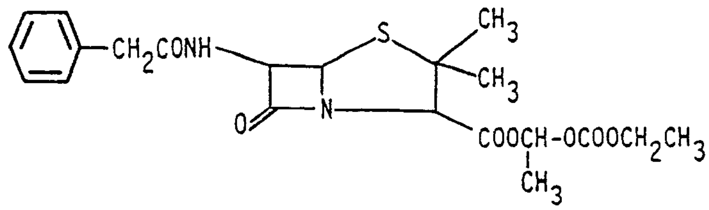

1'-ethoxycarbonyoxy ethyl benzyl penicillinate

$CH_3CH{-}OCOOC_2H_5$
|
Br

1-bromoethyl ethyl carbonate

The process of invention is further illustrated by the following Examples. The preparation of 1-bromoethyl ethyl carbonate, which is not described in published prior art, is also illustrated.

Preparation of 1-bromoethyl ethyl carbonate

A mixture of 354 g (3 mole) diethylcarbonate and 700 ml carbon tetrachloride was stirred and irradiated externally with a 1.5 kw iodine — quartz lamp. The heat from the lamp brought the mixture to reflux. Bromine (412.8 g, 2.58 mole) was added slowly through a dip-pipe below the surface of the mixture over a period of 15—17 hours. The colour of the solution was kept light red throughout the reaction. The temperature in the solution was 84—85°C. Hydrogen bromide evolved copiously during the addition. The reaction mixture was cooled, washed with aqueous sodium bisulfite and dried over magnesium sulfate. Carbon tetrachloride was distilled at atmospheric pressure. The rest was fractionally distilled at 30 mm Hg pressure. The first fraction consisted of unreacted diethylcarbonate, the second fraction boiled at 90—95° (30 mm pressure) was 1-bromoethyl ethyl carbonate. The residue in the pot was mainly bis-(1-bromoethyl)carbonate. The yield was about 52% calculated on bromine.

The following physical data of 1-bromoethyl ethyl carbonate:

Boiling point at 60 mm Hg — 110°

Bromine content 40.6% (theoretical 40.61%)

Density ($D_4^{20}$) 1.4244

Refraction index ($n_D^{20}$) 1.4395

NMR Spectrum: 1.4 ppm 3H triplet J=7Hz
2.0 ppm 3H doublet J=6Hz
4.25 ppm 2H quartet J=7Hz
6.6 ppm 1H quartet J=6Hz

GC analysis showed it to be a pure compound.

Example 1

A mixture of potassium benzyl penicillinate (20 g), sodium bicarbonate (18.15 g), water (2.4 ml) and acetone (60 ml) was heated to 40°C. 1-Bromoethyl ethyl carbonate (21.2 g) was added during 30 minutes. The reaction mixture was stirred for 4.5 hours at 40°. Water (60 ml) was added and the reaction mixture stirred for 1 hour at ca. 30°C. Ethyl acetate (60 ml) was added and the mixture was filtered. The organic phase was separated and washed with a saturated sodium bicarbonate solution and 10% sodium chloride solution. The mixture was dried and the solvent evaporated under reduced pressure. 1'-Ethoxycarbonyloxyethyl benzyl penicillinate was obtained in quantitative yield and 99% purity (assayed by HPLC and mercurometric titration).

Example 2

A mixture of potassium benzyl penicillin (20 g), 1-bromoethyl ethyl carbonate (19.15 g) sodium bicarbonate (18.15 g), tetrabutyl ammonium bromide (1 g) and acetone (60 ml) was heated at 45°C for 2.5 hours. Water (60 ml) was added and the reaction mixture stirred for 1 hour and then ethyl acetate (60 ml) was added. The organic phase was separated, washed with a sodium bicarbonate solution (2 × 60 ml) and a 20% sodium chloride solution.

The organic phase was dried over sodium sulphate and decolourized with charcoal. The solvent was

evaporated under reduced pressure to yield 22.1 g of 1-ethoxy carbonyloxyethyl benzyl penicillinate 99% pure by G.C. and mercurometry (19.1% total yield).

Preparation of bacampicillin

1'Ethoxycarbonyloxy ethyl benzyl penicillinate (100 g), obtained as described in the preceding examples, was dissolved in methylene chloride (375 ml). N,N-Dimethyl aniline (61 ml) was added and the mixture was cooled to −80°C. Phosphorous pentachloride was added in three portions of 31 g taking care that the temperature does not rise above −60°C. The mixture was stirred at −70° for 1,5 hours. Cold methanol (140 ml) was added slowly keeping the temperature below −55°C. Stirring was continued at −70°C for 2 hours. Water (500 ml) and petroleum ether (450 ml) were added and the phases separated. The organic layer was washed with water (500 ml). The combined aqueous fractions were combined and basified to pH 7 with ammonia, keeping the temperature at 10°C or below.

The organic phase was separated and triturated with petroleum ether (3 × 100 ml) and the solvent decanted each time. The residual thick oil was dissolved in methylene chloride (240 ml). Sodium bicarbonate (30 g) and water (3 ml) were added. The mixture was cooled to −5°C. D(-) phenylglycyl chloride hydrochloride (37.2 g) was added and the mixture was stirred for 2 hours at 0°C. The mixture was filtered from solids, methylene chloride was evaporated. Butyl acetate (240 ml) and isopropanol (30 ml) were added. Becampicillin crystallized slowly at 0°C. The solid was filtered, washed and dried. The yield of becampicillin was 68.2 g.

## Claims

1. A process for the preparation of 1'ethoxycarbonyloxy ethyl benzyl penicillinate:

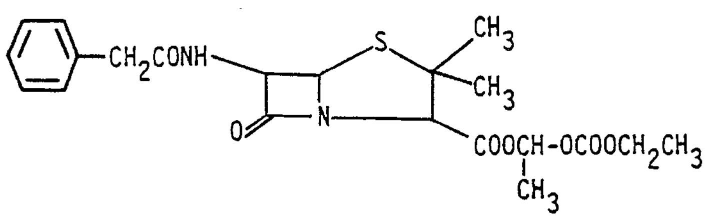

characterized in that benzyl penicillin:

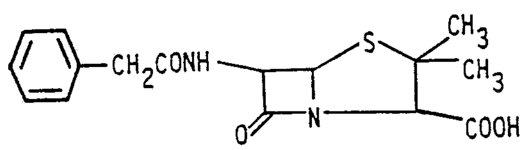

or a salt thereof, is reacted with 1-bromoethyl ethyl carbonate:

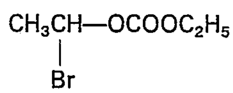

in a non-hydroxylic polar solvent at a temperature of from 20°C to 50°C.

2. A process according to claim 1, characterized in that the potassium salt of benzyl penicillin is reacted with 1-bromoethyl ethyl carbonate in acetone at a temperature of 35°C to 45°C.

3. A process according to claims 1 and 2 characterized in that the reaction is carried out at 40°C.

4. A process according to claims 1—3, characterized in that it is carried out in the presence of a catalytic amount of tetrabutyl ammonium bromide.

5. The use of 1-bromoethyl ethyl carbonate as esterification agent in a process for the preparation of 1'-ethoxycarbonyloxy ethyl benzyl penicillinate as claimed in claims 1—4.

**Patentansprüche**

1. Verfahren zur Herstellung von Ethoxycarbonyloxyethylbenzylpenicillinat

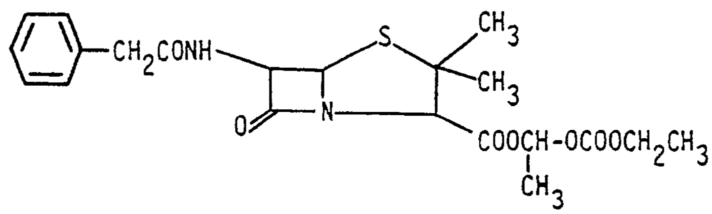

dadurch gekennzeichnet, daß Benzylpenicillin

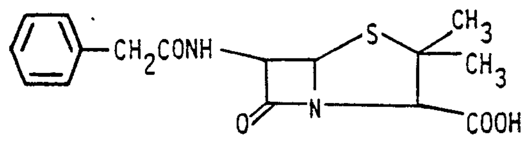

oder ein Salz desselben mit 1-Bromethylethylcarbonat

$$\underset{\displaystyle Br}{CH_3\underset{|}{C}H}-OCOOC_2H_5$$

in einem nicht hydroxylhaltigen polaren Lösungsmittel bei einer Temperatur von 20°C bis 50°C umgesetzt wird.

2. Verfahren nach Anspruch 1, dadurch gekennzeichnet, daß das Kaliumsalz von Benzylpenicillin mit 1-Bromethylethylcarbonat in Aceton bei einer Temperatur von 35°C bis 45°C umgesetzt wird.

3. Verfahren nach den Ansprüchen 1 und 2, dadurch gekennzeichnet, daß die Umsetzung bei 40°C erfolgt.

4. Verfahren nach den Ansprüchen 1 bis 3, dadurch gekennzeichnet, daß es in Gegenwart einer katalytischen Menge von Tetrabutylammoniumbromid durchgeführt wird.

5. Verwendung von 1-Bromethylethylcarbonat als Veresterungsmittel in einem Verfahren zur Herstellung von 1′-Ethoxycarbonyloxyethylbenzylpenicillinat nach den Ansprüchen 1 bis 4.

**Revendications**

1. Un procédé pour la préparation du 1-éthoxycarbonyloxyéthyl pénicillinate de benzyle

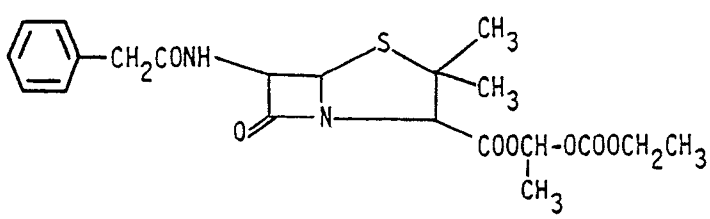

caractérisé en ce que la benzyl-pénicilline:

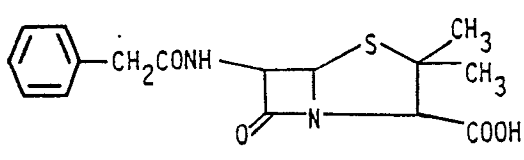

ou un de ses sels, est mise à réagir avec du 1-bromoéthylcarbonate d'éthyle

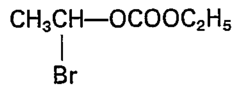

dans un solvant non-hydroxylique polaire à une température de 20°C à 50°C.

2. Un procédé selon la revendication 1, caractérisé en ce que le sel de potassium de benzyl-pénicilline est mis à réagir avec du 1-bromoéthyl-carbonate d'éthyle dans l'acétone à une température de 35°C à 45°C.

3. Un procédé selon les revendications 1 et 2, caractérisé en ce que la réaction est mis en oeuvre à 40°C.

4. Un procédé selon les revendications 1 à 3, caractérisé en ce qu'il est mis en oeuvre en présence d'une quantité catalytique de bromure de tétrabutyl-ammonium.

5. L'utilisation de 1-bromoéthyl-carbonate d'éthyle comme agent d'estérification dans un procédé pour la préparation du 1-éthoxycarbonyloxyéthyl-pénicillinate de benzyle.